# EUROPÄISCHE PATENTSCHRIFT

(11) **EP 1 517 691 B1**
(45) Veröffentlichungstag und Bekanntmachung des Hinweises auf die Patenterteilung: **29.09.2010**
(21) Anmeldenummer: 03808679.9
(22) Anmeldetag: 27.06.2003
(51) Int. Cl.: A61K 31/48, A61K 31/137, A61K 31/4045, A61K 31/428, A61P 17/02, A61P 17/12, A61P 35/00

(54) **VERWENDUNG VON DOPAMIN-REZEPTOR-AGONISTEN ZUR BEHANDLUNG VON HAUTGESCHWÜLSTEN, WARZEN UND NARBEN**
USE OF DOPAMINE RECEPTOR AGONISTS FOR THE TREATMENT OF CUTANEOUS TUMORS, WARTS, AND SCARS
UTILISATION D'AGONISTES DE RECEPTEUR DE DOPAMINE POUR TRAITER DES TUMEURS CUTANEES, DES VERRUES ET DES CICATRICES

(30) Priorität: 01.07.2002 DE 10229456
(43) Veröffentlichungstag der Anmeldung: 30.03.2005
(73) Patentinhaber: Wank, Rudolf, 80469 München (DE)
(72) Erfinder: Wank, Rudolf, 80469 München (DE)
(74) Vertreter: Wibbelmann, Jobst
(86) Internationale Anmeldenummer: PCT/EP2003/006851
(87) Internationale Veröffentlichungsnummer: WO 2004/035055

(56) Entgegenhaltungen:
- EP-A- 0 680 761
- WO-A-99/47133
- US-A- 4 978 332
- US-A- 5 792 748
- US-A1- 2001 049 350

## Beschreibung

Die vorliegende Erfindung betrifft die Verwendung von Dopamin-Rezeptor-Agonisten, insbesonder Dopamin-D₂-Rezeptor-Agonisten, zur lokalen Behandlung von Hautgeschwülsten und Warzen, insbesondere in Kombination mit Dimethylsulfoxid.

Dopamin-Rezeptor-Agonisten, insbesondere Dopamin-D₂-Rezeptor-Agonisten, weisen eine dopaminerge Wirkung auf und werden hauptsächlich zur Behandlung der Parkinson-Krankheit eingesetzt. Die Verabreichung der Wirkstoffe als Anti-Parkinsonmittel erfolgt üblicherweise oral, in Form von Tabletten. Einige der Dopamin-D₂-Rezeptor-Agonisten, wie beispielsweise Bromocriptin, werden auch zur Behandlung von weiteren Erkrankungen wie Hyperprolactinämie, Akromegalie oder Hypophysentumoren eingesetzt. Ferner ist aus der GB-A-2 273 873 eine topische pharmazeutische Präparation, die Bromocriptin als Wirkstoff enthält, zur Verwendung zur Behandlung von Psoriasis bekannt.

Des Weiteren offenbart die US-A-5 792 748 die Behandlung von Melanomen durch Modulierung des Prolaktinspiegels mit systemisch verabreichtem Bromocriptin. Die US 2001/049350 A1 beschreibt zudem die systemische Verwendung von Bromocriptin zur Behandlung von Tumoren mittels photodynamischer Therapie.

Aus der WO 99/47133 A1 ist ferner eine Selegilin enthaltende Präparation zur topischen Behandlung von Wunden, Verbrennungen und lichtgeschädigter Haut bekannt. Die US-A-4 978 332 offenbart ferner die Behandlung von neoplastischen Erkrankungen mit Chemotherapeutika in Kombination mit vasokonstriktiven Substanzen, beispielsweise dem Ergot-Alkaloid Ergotamin. Schließlich beschreibt die EP-A-0 680 761 die lokale Behandlung von Narben mit Verbindungen, wie Vincamicin, die vasokinetische Aktivität besitzen.

Es wurde nun überraschend gefunden, dass topische pharmazeutische Präparationen von Dopamin-Rezeptor-Agonisten zur lokalen Behandlung von Hautgeschwülsten und Warzen geeignet sind.

Die Erfindung betrifft daher die Verwendung eines Dopamin-Rezeptor-Agonisten oder eines pharmazeutisch annehmbaren Salzes davon zur Herstellung einer topischen pharmazeutischen Präparation zur lokalen Behandlung von Hautgeschwülsten und Warzen. Die erfindungsgemäße lokale Applikation von Dopamin-Rezeptor-Agonisten kann zusätzlich durch orale Verabreichung von Dopamin-Rezeptor-Agonisten unterstützt werden.

Unter Hautgeschwülste im Rahmen dieser Erfindung fallen sowohl Hautgeschwülste der Krebsvorstufe wie auch nichtmetastasierende Krebsgeschwülste der Haut. Insbesondere kann es sich dabei um aktinische Keratosen, Basaliome oder Bowneoide handeln.

Zu den Warzen, die im Rahmen der vorliegenden Erfindung behandelt werden können, gehören beispielsweise interdigitale Warzen, plane Warzen, plantare Warzen, vulgäre Warzen oder Kondylome.

Die erfindungsgemäße Verwendung umfasst grundsätzlich alle Dopamin-Rezeptor-Agonisten, bevorzugt die Dopamin-D₂-Rezeptor-Agonisten. Dazu gehören insbesondere Bromocriptin (2-Brom-a-ergocryptin), Pergolid (D-6-n-Propyl-8β-methylmercaptomethylergolin), Selegilin, Ropivinol (4-[2-(Dipropylamino)ethyl]-1,3-dihydro-2H-indol-2-on) , Pramipexol ((S)-4,5,6,7-Tetrahydro-N⁶-propyl-2,6-benzothiazoldiamin) oder Cabergolid (6-Allyl-N-[3-(dimethylamino)-propyl]-N-(ethylcarbamoyl)ergolin-8β-carboxamid). Besonders bevorzugt wird Bromocriptin eingesetzt. Pharmazeutische Präparate dieser Wirkstoffe sind in Tablettenform kommerziell erhältlich.

Gemäß einer bevorzugten Ausführungsform enthält die pharmazeutische Präparation Dimethylsulfoxid (DMSO). Die Kombination von DMSO und einem Dopamin-Rezeptor-Agonisten führt zu unerwarteten synergistischen Effekten bei der Behandlung von Hautgeschwülsten und Warzen. Dimethylsulfoxid ist in großer Reinheit (p.a.-Qualität) kommerziell erhältlich.

Als pharmazeutisch annehmbare Salze kommen sowohl wasserlösliche wie auch schwerwasserlösliche Salze, die dem Fachmann allgemein bekannt sind, in Betracht. Unter die erfindungsgemäßen topischen Präparationen, die ein oder mehrere Dopamin-Rezeptor-Agonisten als Wirkstoff enthalten, und sich zur Applikation auf der Haut eignen, fallen Salben, Pasten, Lotionen, Cremes, Gele oder auch Tinkturen.

Die Herstellung der Präparationen erfolgt in bekannter Weise, wobei die bekannten und üblichen pharmazeutischen Hilfsstoffe sowie sonstige übliche Träger- und Verdünnungsmittel verwendet werden (List et al., Arzneiformenlehre, Wiss. Verlagsges., Stuttgart, 1985; Sucka et al., Pharmazeutische Technologie, Thieme Verlag, Stuttgart, 1978).

Beispielsweise können bei der Herstellung einer Salbe Organogele (z.B. Vaseline, Plastibase), Lipogele (z.B. Bienenwachs), Hydrogele (z.B. Aerosil^{®}, Bentonite, Stärkederivate, Polyacrylsäure, Polyethylenglykole) oder Silicongele als Salbengrundlage verwendet werden. Bevorzugt dient Vaseline als Salbengrundlage, wobei die Salbe noch weitere Materialien wie Fettalkohole, Glycerylmonostearate, Triglyceride, Alkylenglykole, Dimethylsulfoxid und Wasser enthalten kann. Ganz besonders bevorzugt wird als Salbengrundlage die DAC-Basiscreme (Cremor basalis, Deutscher Arzneimittel-Codex) verwendet, die weiße Vaseline, Glycerolmonostearat 60, Cetylalkohol, mittelkettige Triglyceride, Macrogol-1000-glycerolmonostearat, Propylenglykol und Wasser enthält.

Bei der Herstellung von Cremes können die oben angeführten Salbengrundlagen in Kombination mit größeren Mengen Wasser verwendet werden, insbesondere auch Fette und Öle, Wachse, Fettsäuren und Fettsäureester, langkettige Alkohole und Emulgierstoffe.

Zu den Präparaten in Gel-Form gehören Organogele (d.h. Kohlenwasserstoff-gele z.B. Vaseline, Plastibase), Lipogele (z.B. natürliche Fette, Bienenwachs), Hydrogele (z.B. Hydroxypropylcellulose, Hydroxypropylmethylcellulose, Aerosil^{®}, Bentonite, Stärkederivate, Polyacrylsäure, Polyethylenglykole), Silicongele oder Emulsionsgele, die übliche Emulgatoren enthalten wie auch Oleogele, die im wesentlichen aus flüssigem Paraffin mit Polyethylen oder Ölen und Verdickungsmitteln zusammengesetzt sind.

Darüber hinaus ist der Zusatz von Konservierungsmitteln, Stabilisatoren, Puffersubstanzen, Farbstoffen, Antioxidantien usw. möglich.

Zur Herstellung von Tinkturen zur topischen Anwendung kommen beispielsweise Wasser oder physiologisch verträgliche Lösungsmittel wie beispielsweise Alkohole (Ethanol, Propylenglykol oder Polyglykole), Öle oder Paraffine in Frage.

Die erfindungsgemäßen pharmazeutischen topischen Präparationen enthalten den Dopamin-Rezeptor-Agonisten oder ein pharmazeutisch annehmbares Salz davon in einer Menge von 0,1 bis 10 Gew.-%, bezogen auf die pharmazeutische Präparation. Bevorzugt liegt der Wirkstoffgehalt im Bereich von 0,25 - 0,5 Gew.-%.

Gemäß einer bevorzugten Ausführungsform enthält die pharmazeutische Präparation zusätzlich zu dem Dopamin-Rezeptor-Agonisten DMSO. Das DMSO kann in der pharmazeutischen Präparation in 5-20 Gew.-%, bevorzugt 10-15 Gew.-%, bezogen auf die pharmazeutische Präparation, enthalten sein.

Zur Einführung der Dopamin-Rezeptor-Agonisten in die topischen Präparationen gemäß der Erfindung können handelsübliche Wirkstoffzubereitungen verwendet werden. Die Tabletten können dabei in geeigneten Lösungsmitteln, wie beispielsweise Ethanol, gelöst werden. Bromocriptin-Tabletten oder -Kapseln sind beispielsweise erhältlich bei Novartis Pharma (Pravidel^{®}), Ratiopharm (Bromocriptin-Ratiopharm^{®}) und Hexal/Neurohexal (Bromocrel^{®}).

Gemäß einer bevorzugten Ausführungsform liegt die pharmazeutische Präparation für die erfindungsgemäße Verwendung in Form einer Salbe vor, die Bromocriptin oder ein pharmazeutisch annehmbares Salz davon in einer Menge von 0,25-0,5 Gew.-%, bezogen auf die pharmazeutische Präparation, enthält.

Ohne dass beabsichtigt wird, auf eine bestimmte Theorie zur Erklärung der feststellbaren Wirkung von Dopamin-Rezeptor-Agonisten, insbesondere in Kombination mit DMSO, festgelegt zu werden, werden die im Folgenden erläuterten Wirkungsmechanismen in Betracht gezogen.

Aus in vitro Experimenten ist die Hemmung des Fibroblastenwachstums durch den Dopamin-D₂-Rezeptor-Agonisten Bromocriptin bekannt (Pawlikowski M., Stepien H., Cell Tissues Res 1979; 202(1):165-169; Pawlikowski M. et al, J Neural Transm 1983; 56(1):91-95). Die vorstehend aufgeführten Hautgeschwülste weisen die Gemeinsamkeit auf, dass sie fibroblastenreich sind und dass eine Induktion durch humane Papillomviren diskutiert wird. Neuere Untersuchungen ergaben, dass humane Papillomviren primär gutartige Tumore der Haut und Schleimhäute, wie Warzen und Kondylome, induzieren (Bojanovsky A., "Erreger bedingter Krankheiten", Hrsg. Jung E.G., Dermatologie, Hippocrates Verlag Stuttgart, 1998, S. 136-139). Hierzu zählen plane Warzen, vulgäre Warzen, plantare Warzen, Feigwarzen (Kondylome). Aus den planen Warzen können sich das Bowneoid oder die Verrucosis generalisata entwickeln, mit einem Potential zur malignen Transformation als Spinaliom. Es scheint, dass die Dopamin-Rezeptor-Agonisten, wie beispielsweise Bromocriptin, einen Doppeleffekt aufweisen können, nämlich als Hemmer des Fibroblastenwachstums und als Hemmer des Keratinozytenwachstums. Aus einer weiteren Studie ging hervor, dass Bromocriptin auch zu einer Modulierung des MDR (multi-drug resistance) assoziierten P-Glykoproteins führen kann (Orlowski et al, Biochem Biophys Res Commun 1998; 244:481-488).

Die These, dass Bromocriptin vermutlich apoptotisch auf Fibroblasten wirkt, wurde auch durch die Beobachtung einer Patientin unterstützt, die die Bromocriptincreme auf eine wulstartige Narbe (Keloid) applizierte. Das 4 cm große Narbenkeloid entstand nach einer autologen Hauttransplantation. Narben wachsen nicht mehr und bestehen hauptsächlich aus Kollagenfasern und ihren Produzenten, den Fibroblasten. Nach vier Wochen Bromocriptinbehandlung war die Narbe kaum mehr sichtbar. Es kann sich dabei nicht um eine Wirkung auf Keratinozyten handeln. Fraglich ist, ob neben der Wirkung auf die Fibroblasten noch eine direkte Wirkung auf die Kollagenfasern selbst erzielt wird.

Dimethylsulfoxid (DMSO) ist ein Resorptionsbeschleuniger und Antiphlogistikum, und weist auch geringgradige anästhetische Eigenschaften auf. Insbesondere scheint aber DMSO auch als Inhibitor des Keratinozytenwachstums zu wirken. Dies könnte eine Erklärung für den synergistischen Effekt bei der Verwendung von DMSO in Kombination mit einem Dopamin-Rezeptor-Agonisten, insbesondere in Bezug auf die Hemmung des Keratinozytenwachstums, darstellen. Möglich wäre auch, dass DMSO, das vor allem zur Penetrationsverbesserung eingesetzt wird, durch Bromocriptin über den P-Glykoprotein-Membrantransporter an den Tumorepithelzellen eine eigenständige Wirkung entfaltet.

Die pharmazeutische Präparation in topischer Form wird mehrmals täglich, in der Regel zwei bis vier Mal pro Tag, auf die betroffenen Hautareale aufgetragen. Je nach Bedarf kann die Häufigkeit der Applikation angepasst werden. Die lokale Behandlung von Hautgeschwülsten und Warzen mit der erfindungsgemäßen Präparation kann zusammen mit einer auf die Erkrankung abgestimmten medikamentösen Behandlung erfolgen. Dazu gehören insbesondere Therapien mit aktivierten Immunzellen, wie sie in der WO 99/50393 beschrieben worden sind. Gemäß einer bevorzugten Ausführungsform wird die topische Präparation durch eine Zusatztherapie mit oralen Präparationen von Dopamin-Rezeptor-Agonisten unterstützt.

Im Folgenden wird die Erfindung anhand von Beispielen näher erläutert.

### Herstellung einer Bromocriptin-Salbe

Als Salbengrundlage wurde die Basiscreme DAC (Cremor basalis, Deutscher Arzneimittel-Codex) mit folgender Zusammensetzung eingesetzt:

| auf 100 g Zubereitung: | |
|---|---|
| Glycerolmonostearat 60 | 4,0 g |
| Cetylalkohol | 6,0 g |
| Mittelkettige Triglyceride | 7,5 g |
| Weiße Vaseline | 25,5 g |
| Macrogol-1000-glycerolmonostearat | 7,0 g |
| Propylenglycol | 10,0 g |
| Wasser | 40 g |

Für die Herstellung der Salbe wurden 77 g Basiscreme DAC, 12 g Dimethylsulfoxid und 250 mg Bromocriptin, in 11 g Ethanol gelöst, eingesetzt.

### Patientenstudien

Bei einer 28-jährigen Patientin mit einem Bowneoid im Genitalbereich, die leider bei der Anwendung von aktivierten Immunzellen Migräneanfälle bekam, wurden aktivierte Immunzellen in niedriger Dosierung zusammen mit lokal aufgetragener Bromocriptinsalbe verwendet. Die vorher bereits 6 Monate durchgeführte Zelltherapie, die das Tumorwachstum verhinderte, jedoch nicht zur Eliminierung des Tumors führte, konnte nach zwei Wochen zusätzlicher Bromocriptin-Salbentherapie (aus obigem Beispiel) nach Verschwinden des Tumors beendet werden. Auch nach dreijähriger Beobachtungszeit trat kein Rezidiv auf.

Ein 60-jähriger Patient mit einem kleinzelligen Bronchialcarcinom, der seit 5 Jahren mit aktivierten Immunzellen behandelt wurde, hatte außerdem aktinische Keratosen (eine Krebsvorstufe) und multiple Basaliome (ein Hautkrebs, der sehr selten metastasiert) am Rumpf und im Gesicht, die mindestens 2 x jährlich operativ entfernt wurden. Die Zelltherapie hatte das Wachstum der Basaliome deutlich reduziert, die meisten verschwanden, einige blieben in einer Art Ruhestatus, ebenfalls die aktinischen Keratosen. Nach ca. 6 Monaten zusätzlicher Bromocriptin-Salbentherapie (aus obigem Beispiel) war ca. die Hälfte der Hautgeschwülste verschwunden, teilweise auch die aktinischen Keratosen, die offensichtlich langsamer absterben als die Basaliome. Die Narbengebiete nach Basaliomexcision, die früher Ort der wiederholten Resektion waren, werden vom Chirurgen halbjährlich inspiziert. Chirurgische Interventionen an der Haut wurden seit zwei Jahren nicht mehr durchgeführt.

Ein 85-jähriger Patient, der einmal im Monat zur Behandlung rezidivierender Infekte kam, klagte über Warzen zwischen den Zehen. Sie wurde u. a. operativ entfernt, rezidivierten immer wieder und waren sehr schmerzhaft. Das Problem der erfolglosen Behandlung bzw. ihrer zeitlich begrenzten Wirkung und ihrer Schmerzhaftigkeit sind bekannt. Die Warzen verschwanden nach Bromocriptin-Salbentherapie (aus obigem Beispiel) nach 72 Stunden. Sicherheitshalber wurde die Therapie 1 Woche durchgeführt, der Patient ist seit einem Jahr rezidivfrei geblieben.

Eine 72-jährige Patientin wurde bereits mehrfach im Gesichtsbereich, vor allem am Nasenrücken/Nasenflügel jedes Jahr operiert. Nach vier Wochen Bromocriptin-Salbentherapie (aus obigem Beispiel) konnte die bereits geplante Operation wegen des deutlichen Tumorrückgangs abgesagt werden. Nach ca. 8 Monaten war die früher deutliche Schwellung und Rötung des Bowneoids nur mehr minimal vorhanden, auch neu an anderen Stellen auftretende Bowneoide sprechen auf die Creme an. Obwohl das Bowneoid noch nicht eliminiert ist, ist das Ergebnis sehr zufriedenstellend, da keine operative Intervention erforderlich ist und auch das kosmetische Ergebnis das der Chirurgie deutlich übertrifft. Möglicherweise wäre
eine zusätzliche Therapie mit aktivierten Immunzellen oder zusätzlich orales Bromocriptin schneller erfolgreich, jedoch ist die lokale hohe Konzentrierung von Bromocriptin sicher ein essentieller Faktor der Behandlung. Orales Bromocriptin hat einige Nebenwirkungen (bis hin zu Gehirnblutungen), die durch lokale Therapie vermieden werden kann. Es sei erwähnt, dass bei Prolactin-bildenden Hypophysentumoren die orale Bromocriptin-Therapie nicht abgesetzt werden sollte, da anderenfalls die Entstehung eines Rezidivs auftreten kann.

Bei einer 55-jährigen Patientin mit Warzen zwischen den Füßen verschwanden die Warzen nach etwa 2 Wochen der Salbenanwendung mit Bromocriptin (aus obigem Beispiel). Ein Rezidiv trat nach etwa 1 Jahr auf, was wahrscheinlich eine Folge eines Fehlers im Behandlungsschema war. Nach korrektem Behandlungsschema (morgens und abends, im Einzelfall kann eine seltenere oder häufigere Anwendung angebracht sein) trat kein Rezidiv mehr auf (seit über zwei Jahren).

Eine 32-jährige Patientin mit einer vulgären Warze konnte durch Anwendung der Bromocriptin-Salbentherapie (aus obigem Beispiel) über 1 Woche erfolgreich behandelt werden. Seit 1 Jahr ist die Patientin ohne Rezidiv.

Bei einem 61-jährigen Patienten mit einer planen Warze im Schläfenbereich verschwand die Warze nach Bromocriptin-Salbentherapie (aus obigem Beispiel) nach einigen Wochen. Bei diesem Patienten war zunächst eine erfolgreiche Behandlung nach 3 Wochen erkennbar, allerdings trat nach 2 Monaten ein Rezidiv mit zusätzlichen angrenzenden 'Tochterwarzen' auf. Diese verschwanden innerhalb von 5 Tagen durch Bromocriptin-Salbentherapie, die ursprüngliche Warze im Schläfenbereich ist nahezu verschwunden, der Patient ist seit 4 Monaten rezidivfrei.

Die erfindungsgemäße bromocriptinhaltige Salbe führte innerhalb von kurzer Zeit zu einem deutlichen Rückgang von Hautgeschwülsten und Warzen und eröffnet somit neue und effiziente Ansätze bei der Behandlung solcher und verwandter Erkrankungen.

## Patentansprüche

1. Verwendung eines Dopamin-Rezeptor-Agonisten oder eines pharmazeutisch annehmbaren Salzes davon zur Herstellung einer topischen pharmazeutischen Präparation zur lokalen Behandlung von Hautgeschwülsten und Warzen.

2. Verwendung gemäß Anspruch 1, **dadurch gekennzeichnet, dass** der Dopamin-Rezeptor-Agonist ein Dopamin-D₂-Rezeptor-Agonist ist.

3. Verwendung gemäß Anspruch 1 oder 2, **dadurch gekennzeichnet, dass** der Dopamin-Rezeptor-Agonist Bromocriptin, Pergolid, Ropirinol, Pramipexol oder Cabergolid ist.

4. Verwendung gemäß einem der Ansprüche 1 bis 3, **dadurch gekennzeichnet, dass** es sich bei den Hautgeschwülsten um Hautgeschwülste der Krebsvorstufe oder nichtmetastasierende Krebsgeschwülste der Haut handelt.

5. Verwendung gemäß einem der Ansprüche 1 bis 4, **dadurch gekennzeichnet, dass** sich bei den Hautgeschwülsten um aktinische Keratosen, Basaliome oder Bowneoide handelt.

6. Verwendung gemäß einem der Ansprüche 1 bis 3, **dadurch gekennzeichnet, dass** es sich bei den Warzen um interdigitale Warzen, plane Warzen, plantare Warzen, vulgäre Warzen oder Kondolyme handelt.

7. Verwendung gemäß einem der Ansprüche 1 bis 6, **dadurch gekennzeichnet, dass** die pharmazeutische Präparation einen Dopamin-Rezepor-Agonisten oder ein pharmazeutisch annehmbares Salz davon in einer Menge von 0,1 bis 10 Gew.-%, bezogen auf die pharmazeutische Präparation, enthält.

8. Verwendung gemäß Anspruch 7, **dadurch gekennzeichnet, dass** die pharmazeutische Präparation einen Dopamin-Rezepor-Agonisten oder ein pharmazeutisch annehmbares Salz davon in einer Menge von 0,25 bis 0,5 Gew.-%, bezogen auf die pharmazeutische Präparation, enthält.

9. Verwendung gemäß Anspruch 8, **dadurch gekennzeichnet, dass** die pharmazeutische Präparation Bromocriptin oder ein pharmazeutisch annehmbares Salz davon in einer Menge von 0,25 bis 0,5 Gew.-%, bezogen auf die pharmazeutische Präparation, enthält.

10. Verwendung gemäß einem der Ansprüche 1 bis 9, **dadurch gekennzeichnet, dass** die pharmazeutische Präparation in Form einer Salbe, Paste, Lotion, Creme oder eines Gels vorliegt.

11. Verwendung gemäß einem der Ansprüche 1 bis 10, **dadurch gekennzeichnet, dass** die pharmazeutische Präparation übliche pharmazeutische Hilfsstoffe, Trägermittel und/oder Verdünnungsmittel enthält.

12. Verwendung gemäß einem der vorhergehenden Ansprüche, **dadurch gekennzeichnet, dass** die pharmazeutische Präparation lokal auf die betroffenen Hautareale ein oder mehrmals täglich aufgetragen wird.

13. Verwendung gemäß einem der vorhergehenden Ansprüche, **dadurch gekennzeichnet, dass** die Verwendung der pharmazeutischen Präparation zusammen mit einer auf die Erkrankung abgestimmten medikamentösen Behandlung erfolgt.

14. Verwendung gemäß einem der vorhergehenden Ansprüche, **dadurch gekennzeichnet, dass** die Verwendung der topischen pharmazeutischen Präparation zusammen mit einer oralen Zusatztherapie eines Dopamin-Rezeptor-Agonisten erfolgt.

15. Verwendung gemäß einem der vorhergehenden Ansprüche, **dadurch gekennzeichnet, dass** die pharmazeutische Präparation Dimethylsulfoxid enthält.

16. Verwendung gemäß Anspruch 15, **dadurch gekennzeichnet, dass** die pharmazeutische Präparation 5-20 Gew.-% Dimethylsulfoxid, bevorzugt 10-15 Gew.-%, bezogen auf die pharmazeutische Präparation, enthält.

## Claims

1. Use of a dopamine receptor agonist or a pharmaceutically acceptable salt thereof for producing a topical pharmaceutical preparation for the local treatment of cutaneous tumours and warts.

2. Use according to Claim 1, **characterised in that** the dopamine receptor agonist is a dopamine D₂ receptor agonist.

3. Use according to Claim 1 or 2, **characterised in that** the dopamine receptor agonist is bromocriptine, pergolide, ropirinole, pramipexole or cabergolide.

4. Use according to one of Claims 1 to 3, **characterised in that** in the case of the cutaneous tumours they are cutaneous tumours of the preliminary stage of cancer or non-metastasising carcinomas of the skin.

5. Use according to one of Claims 1 to 4, **characterised in that** in the case of the cutaneous tumours they are actinic keratoses, basalioma or bowenoids.

6. Use according to one of Claims 1 to 3, **characterised in that** in the case of the warts they are interdigital warts, plane warts, plantar warts, vulgar warts or condyloma.

7. Use according to one of Claims 1 to 6, **characterised in that** the pharmaceutical preparation contains a dopamine receptor agonist or a pharmaceutically acceptable salt thereof in a quantity of from 0.1 wt.% to 10 wt.%, relative to the pharmaceutical preparation.

8. Use according to Claim 7, **characterised in that** the pharmaceutical preparation contains a dopamine receptor agonist or a pharmaceutically acceptable salt thereof in a quantity of from 0.25 wt.% to 0.5 wt.%, relative to the pharmaceutical preparation.

9. Use according to Claim 8, **characterised in that** the pharmaceutical preparation contains bromocriptine or a pharmaceutically acceptable salt thereof in a quantity of from 0.25 wt.% to 0.5 wt.%, relative to the pharmaceutical preparation.

10. Use according to one of Claims 1 to 9, **characterised in that** the pharmaceutical preparation is present in the form of an ointment, a paste, a lotion, a creme or a gel.

11. Use according to one of Claims 1 to 10, **characterised in that** the pharmaceutical preparation contains conventional adjuvants, excipients and/or diluents.

12. Use according to one of the preceding claims, **characterised in that** the pharmaceutical preparation is applied locally onto the affected cutaneous areas once or several times a day.

13. Use according to one of the preceding claims, **characterised in that** the use of the pharmaceutical preparation is undertaken together with a medicinal treatment that is matched to the disease.

14. Use according to one of the preceding claims, **characterised in that** the use of the topical pharmaceutical preparation is undertaken together with an oral adjuvant therapy involving a dopamine receptor agonist.

15. Use according to one of the preceding claims, **characterised in that** the pharmaceutical preparation contains dimethyl sulfoxide.

16. Use according to Claim 15, **characterised in that** the pharmaceutical preparation contains 5-20 wt.% dimethyl sulfoxide, preferably 10-15 wt.%, relative to the pharmaceutical preparation.

## Revendications

1. Utilisation d'un agoniste de récepteur de dopamine ou d'un sel pharmaceutiquement acceptable de celui-ci pour la production d'une préparation pharmaceutique topique pour le traitement local des tumeurs cutanées et des verrues.

2. Utilisation selon la revendication 1, **caractérisée en ce qu'**un agoniste de récepteur de dopamine D₂ est utilisé comme agoniste de récepteur de dopamine.

3. Utilisation selon la revendication 1 ou 2, **caractérisée en ce qu'**il est utilisé de la bromocriptine, du pergolide, du ropirinole, du pramipexole ou de la cabergoline comme agoniste de récepteur de dopamine.

4. Utilisation selon l'une des revendications 1 à 3, **caractérisée en ce qu'**il s'agit, concernant les tumeurs cutanées, de tumeurs précancéreuses ou de tumeurs cancéreuses non métastasantes de la peau.

5. Utilisation selon l'une des revendications 1 à 4, **caractérisée en ce qu'**il s'agit, concernant les tumeurs cutanées, de kératoses actiniques, de basaliomes ou de bowenoides.

6. Utilisation selon l'une des revendications 1 à 3, **caractérisée en ce qu'**il s'agit, concernant les verrues, de verrues interdigitales, de verrues plates, de verrues plantaires, de verrues vulgaires ou de condylomes.

7. Utilisation selon l'une des revendications 1 à 6, **caractérisée en ce que** la préparation pharmaceutique contient un agoniste de récepteur de dopamine ou un sel pharmaceutiquement acceptable de celui-ci dans une quantité comprise entre 0,1 et 10 % en poids par rapport à la préparation pharmaceutique.

8. Utilisation selon la revendication 7, **caractérisée en ce que** la préparation pharmaceutique contient un agoniste de récepteur de dopamine ou un sel pharmaceutiquement acceptable de celui-ci dans une quantité comprise entre 0,25 et 0,5 % en poids par rapport à la préparation pharmaceutique.

9. Utilisation selon la revendication 8, **caractérisée en ce que** la préparation pharmaceutique contient de la bromocriptine ou un sel pharmaceutiquement acceptable de celui-ci dans une quantité comprise entre 0,25 et 0,5 % en poids par rapport à la préparation pharmaceutique.

10. Utilisation selon l'une des revendications 1 à 9, **caractérisée en ce que** la préparation pharmaceutique se présente sous la forme d'un onguent, d'une pâte, d'une lotion, d'une crème ou d'un gel.

11. Utilisation selon l'une des revendications 1 à 10, **caractérisée en ce que** la préparation pharmaceutique contient des adjuvants, des supports et/ou des diluants pharmaceutiques courants.

12. Utilisation selon l'une des revendications précédentes, **caractérisée en ce que** la préparation pharmaceutique est appliquée localement une fois ou plusieurs fois par jour sur les aires cutanées concernées.

13. Utilisation selon l'une des revendications précédentes, **caractérisée en ce que** l'utilisation de la préparation pharmaceutique est assortie d'un traitement médicamenteux déterminé en fonction de la maladie à traiter.

14. Utilisation selon l'une des revendications précédentes, **caractérisée en ce que** l'utilisation de la préparation pharmaceutique topique est assortie à un agoniste de récepteur de dopamine comme thérapie supplémentaire par voie orale.

15. Utilisation selon l'une des revendications précédentes, **caractérisée en ce que** l'utilisation de la préparation pharmaceutique contient du diméthylsulfoxyde.

16. Utilisation selon la revendication 15, **caractérisée en ce que** la préparation pharmaceutique contient entre 5 et 20 % en poids de diméthylsulfoxyde, de préférence entre 10 et 15 % en poids, par rapport à la préparation pharmaceutique.
